# EUROPEAN PATENT SPECIFICATION

(11) **EP 0 614 901 B1**
(45) Date of publication and mention of the grant of the patent: **12.08.1998**
(21) Application number: 94103674.1
(22) Date of filing: 10.03.1994
(51) Int. Cl.: C07F 9/50, C07F 15/00, C07C 45/50, C07F 9/6574, C07F 9/6568

(54) **Phosphine compound and transition metal-phosphine complex containing the same as ligand**
Phosphinverbindung und diese als Ligand enthaltender Übergangsmetall-Phosphinkomplex
Composé de phosphine et complexe métal de transition-phosphine contenant le même ligand

(30) Priority: 12.03.1993 JP 52538/93
(43) Date of publication of application: 14.09.1994
(73) Proprietor: MITSUBISHI GAS CHEMICAL COMPANY, INC., Minato-ku, Tokyo (JP); Takasago International Corporation, Tokyo 108 (JP)
(72) Inventor: Takaya, Hidemasa, Kusatsu-shi, Shiga (JP); Sakai, Nozomu, Kobe-shi, Hyogo (JP); Tamao, Kyoko, Beru Mezon kamogawa 303, Kamikyo-ku, Kyoto-shi, Kyoto (JP); Mano, Satoshi, Chiaki-cho, Ichinomiya-shi, Aichi (JP); Kumobayashi, Hidenori, c/o Takasago Int. Corp., Hiratsuka-shi, Kanagawa (JP); Tomita, Tetsu, Katsushika-ku, Tokyo (JP)
(74) Representative: Kraus, Walter, Dr.

(56) References cited:
- EP-A- 0 104 375
- EP-A- 0 503 884
- WO-A-93/03839
- US-A- 5 171 892
- JOURNAL OF THE AMERICAN CHEMICAL SOCIETY vol. 115, no. 15 , 28 July 1993 , WASHINGTON, DC US pages 7033 - 7034 NOZOMU SAKAI 'Highly Enantioselective Hydroformylation of Olefins Catalized by New Phosphinephosphite-Rh(I) Complexes'

## Description

### BACKGROUND OF THE INVENTION

### Field of the Invention:

The present invention relates to a novel phosphine compound and a transition metal complex which contains this compound as a ligand and is useful as a catalyst for a variety of asymmetric synthetic reactions.

### Description of the Background Art:

Many transition metal complexes have heretofore been used as catalysts for organic synthetic reactions. In particular, noble metal complexes are expensive, but safe and easy to handle, and so many synthetic researches making use of them as catalysts have been made. A great number of reports that organic synthetic reactions said to be infeasible by former methods or processes were made possible have been presented one after another.

Among others, complexes obtained by coordinating an optically active tertiary phosphine to a transition metal such as rhodium, palladium, ruthenium or nickel often have excellent performance as catalysts for asymmetric synthetic reactions. In order to enhance the performance of these catalysts, a number of phosphine compounds having a special structure have been developed to date ["Organometallic Chemistry", Kagaku Sosetsu (The Elements of Chemistry), 32, edited by The Chemical Society of Japan, 237-238 (1982)].

Asymmetric hydroformylation reactions making use of a transition metal-optically active phosphine complex have been known. More specifically, there are described an asymmetric hydroformylation reaction making use of a rhodium complex containing, as a ligand, optically active 2,3-O-isopropylidene-2,3-dihydroxy-1,4-bis(diphenylphosphino)butane (hereinafter called DIOP) in Journal of Organic Chemistry, 46, 4422-4427 (1981); the asymmetric hydroformylation reaction of olefins making use of a rhodium complex containing, as a ligand, an optically active bidentate phosphine (DIOP or the like) in Bulletin of The Chemical Society of Japan, 52, 2605-2608 (1979); and the asymmetric hydroformylation reaction of methyl α-acetamidoacrylate making use of a rhodium complex containing, as a ligand, an optically active bidentate phosphine, e.g., DIOP in Tetrahedron Asymmetry, 10, 693-696 (1990).

As a ligand composed of an optically active tertiary phosphite, a bis (triarylphosphite) having an optically active BINAP structure is described in, for example, Tetrahedron Asymmetry, 3, 583-586 (1992), wherein BINAP means 2,2'-bis(diphenylphosphino)-1,1'-binaphthyl. The asymmetric hydroformylation reaction of vinyl acetate making use of a rhodium complex containing this compound as a ligand is described therein.

### SUMMARY OF THE INVENTION

As described above, many particular phosphine compounds have heretofore been developed for providing catalysts having higher performance as catalysts for asymmetric synthetic reactions. These compounds are not always fully satisfactory from the viewpoint of selectivity, conversion, catalytic activity, optical purity and the like though may vary according to the substrate and reaction intended. It is therefore an object of the present invention to provide a novel phosphine compound which permits the provision of complexes having high catalytic ability compared with the conventionally-known catalysts.

In view of the foregoing circumstances, the present inventor has carried out an extensive investigation with a view toward solving the above problem involved in the conventional catalysts. As a result, it has been found that a transition metal complex containing, as a ligand, a phosphine compound having an optically active binaphthol skeleton is remarkably superior in optical purity and selectivity in asymmetric synthesis to the conventionally-known optically active phosphine-transition metal complexes, thus leading to completion of the present invention.

In an aspect of the present invention, there is thus provided a phosphine compound represented by the following formula (1): 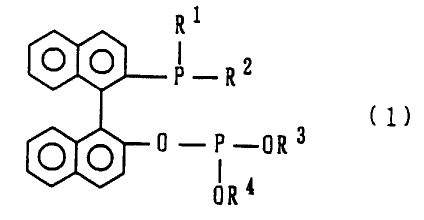 wherein R¹ and R² are identical with or different from each other and mean individually a phenyl group which may be substituted by a halogen atom or lower alkyl group, or denote together a divalent hydrocarbon group, R³ and R⁴ are identical with or different from each other and mean individually a lower alkyl group or a phenyl group which may be substituted by a halogen atom, lower alkyl group or lower alkoxy group, or denote together a divalent hydrocarbon group.

In another aspect of the present invention, there is also provided a transition metal-phosphine complex containing, as a ligand, the phosphine compound as described above.

In a further aspect of the present invention, there is provided a rhodium-phosphine complex represented by the following formula (2-a) or (2-b):

[Rh(L)(Y)(X)] (2-a)

[Rh(L)(Y)](X) (2-b)

wherein L means the phosphine compound as described above, Y denotes carbon monoxide, a monodentate or bidentate neutral ligand having lower coordinating strength than that of carbon monoxide, olefin or diene, and X stands for a hydrogen atom, monovalent anion or triphenylphosphine, or X and Y mean together a β-diketonato.

When the transition metal-phosphine complex according to the present invention is used as a catalyst for asymmetric synthesis, an intended product having a desired absolute configuration can be obtained in a high optical purity at a high yield.

The above and other objects, features and advantages of the present invention will become apparent from the following description and the appended claims.

### DETAILED DESCRIPTION OF THE INVENTION AND PREFERRED EMBODIMENTS

In the present invention, the term "lower alkyl group" means a linear or branched alkyl group having 1-6 carbon atoms, such as a methyl, ethyl, propyl, isopropyl, butyl, isobutyl, sec-butyl, tert-butyl, pentyl or hexyl group. The term "halogen atom" means a chlorine, bromine, iodine or fluorine atom, while the term "lower alkoxy group" means a linear or branched alkoxy group having 1-6 carbon atoms, such as a methoxy, ethoxy, propoxy, isopropoxy, butoxy, isobutoxy, sec-butoxy or tert-butoxy group.

Besides, the term "divalent hydrocarbon group" as used herein means a biarylene group such as a biphenyl group which may have a substituent group, a binaphthyl group which may have a substituent group, a biphenanthryl group which may have a substituent group or an bianthryl group which may have a substituent group, or a linear or branched, saturated or unsaturated alkylene group having 2-7 carbon atoms, such as a tetramethylene, pentamethylene, 1,4-dimethyltetramethylene, 1,3-butadienylene or 1,4-dimethyl-1,3-butadienylene group. Examples of the substituent group substitutable on the bisarylene group include lower alkyl groups, halogen atoms and lower alkoxy groups.

As examples of the transition metal, may be mentioned rhodium, ruthenium, palladium, iridium, platinum, cobalt, nickel, titanium and the like. A rhodium-phosphine complex where the transition metal is rhodium is represented by the following formula (2-a) or (2-b):

[Rh(L)(Y)(X)] (2-a)

[Rh(L)(Y)](X) (2-b)

wherein L means the phosphine compound according to this invention, Y denotes carbon monoxide, a monodentate or bidentate neutral ligand having lower coordinating strength than that of carbon monoxide, olefin or diene, and X stands for a hydrogen atom, monovalent anion or triphenylphosphine, or X and Y mean together a β-diketonate.

The phosphine compound (1) according to the present invention is prepared in accordance with, for example, a process represented by the following reaction scheme: 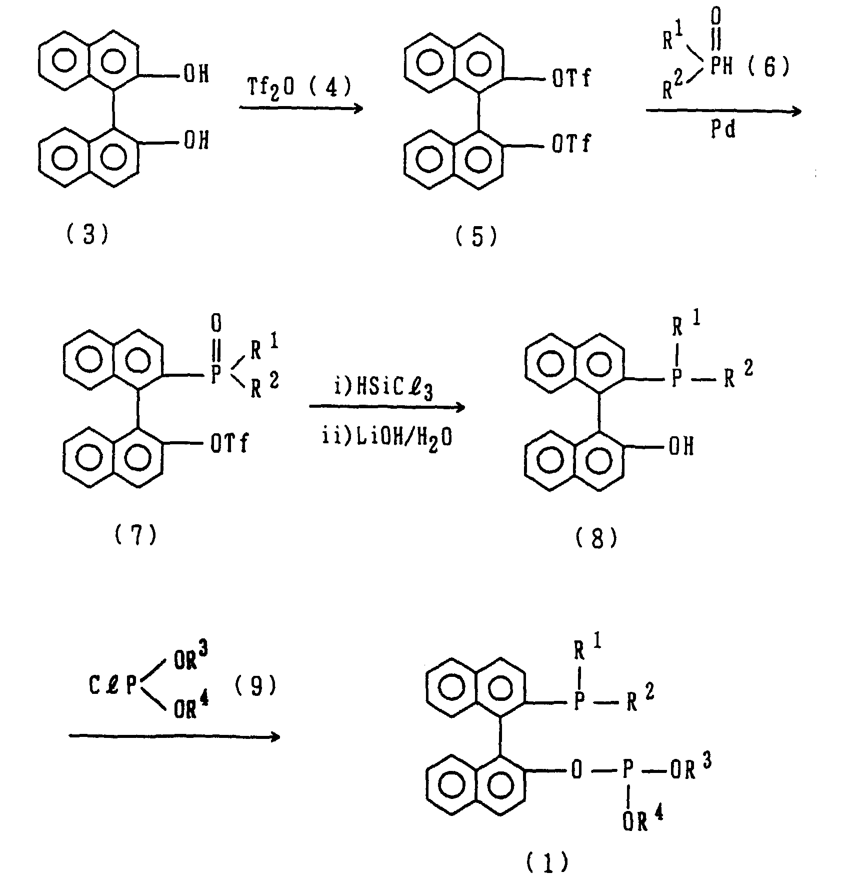 wherein Tf means a trifluoromethanesulfonyl group, and R¹, R², R³ and R⁴ have the same meaning as defined above.

More specifically, following Tetrahedron Letters, 31, 6321-6324 (1990), trifluoromethanesulfonic anhydride (4) is reacted with 1,1-bi-2-naphthol (3) to form 2,2'-bis(trifluoromethanesulfonyloxy)-1,1'-binaphthyl (5). This compound (5) is reacted with phosphine oxide (6) in the presence of a palladium catalyst to form 2-phosphinyl-2'-trifluoromethanesulfonyloxy-1,1'-binaphthyl (7). The compound (7) is then reduced in the presence of triethylamine, followed by hydrolysis of the reduction product to form 2-phosphino-2'-hydroxy-1,1'-binaphthyl (8). Further, this compound (8) is reacted with chlorophosphine (9) in the presence of triethylamine, whereby the phosphine compound (1) of the present invention is prepared.

The optically active 1,1'-bi-2-naphthol used as a starting material in the present invention can be easily synthesized in a high optical purity at a high yield in accordance with the process disclosed in J. Org. Chem., 53, 3607 (1988) and Japanese Patent Application Laid-Open No. 13063/1989. More specifically, it is synthesized by forming a clathrate complex only from optically active O,O'-dimethyl-N,N'-tetramethyltartaric amide synthesized using natural or synthetic tartaric acid as a raw material and one of optically active substances of racemic binaphthol, and then decomposing the clathrate complex.

Besides, the transition metal-phosphine complex (2) according to the present invention can be prepared in accordance with any known method. A description will hereinafter be made taking the case of rhodium.

For example, [Rh(COD)₂]ClO₄ prepared from [Rh(COD)(Cl)]₂ (COD: 1,5-cyclooctadiene) and AgClO₄ in accordance with the process described in J. Am. Chem. Soc., 93, 3089 (1971) is reacted with the phosphine compound (1) (L) according to the present invention, thereby obtaining [Rh(L)(COD)]ClO₄.

Further, [Rh(L)(CO)Cl] is obtained by reacting the phosphine compound (1) of this invention with commercially-available [Rh(CO)₂Cl]₂ at room temperature in methylene chloride in accordance with the process described in Inorg. Synth., 8, 214-217 (1966).

For the binaphthyl skeleton of the phosphine compound (1) according to the present invention, optically active substances and racemic modification exist. All the compounds are embraced in the scope of the present invention.

In this invention, examples of the rhodium-phosphine complex include the following compounds. In the following compounds, L and COD have the same meaning as defined above, NBD means norbornadiene, Ph denotes a phenyl group, acac stands for acetylacetonato, and M is rhodium.

[M(L)(C₂H₄Cl], [M(L)(C₂H₄)Br], [M(L)(C₂H₄F], [M(L)(C₂H₄)I], [M(L)(C₂H₄CN], [M(L)(CO)Cl], [M(L)(CO)Br], [M(L)(CO)F], [M(L)(CO)I], [M(L)(CO)CN], [M(L)(COD)]Cl, [M(L)(COD)]Br, [M(L)(COD)]F, [M(L)(COD)]I, [M(L)(COD)]ClO₄, [M(L)(COD)]BF₄, [M(L)(COD)]PF₆, [M(L)(COD)]CN, [M(L)(COD)]OCOCH₃, [M(L)(NBD)]Cl, [M(L)(NBD)]Br, [M(L)(NBD)]F, [M(L)(NBD)]I, [M(L)(NBD)]ClO₄, [M(L)(NBD)]BF₄, [M(L)(NBD)]PF₆, [M(L)(NBD)]CN, [M(L)(NBD)]OCOCH₃, [M(L)(CO)₂]H, [M(L)(CO)₂(PPh₃)₂], [M(L)(CO)₃(PPh₃)], [M(L)(pyridine)Cl], [M(L)(pyridine)Br] and [M(L)(acac)].

When the transition metal-phosphine complex according to the present invention is used as a catalyst for the asymmetric hydroformylation reaction of, for example, an olefin, an intended product can be obtained in a high optical purity which has been unable to be reached so far. Besides, if one of the optically active phosphine compounds of this invention, which respectively have optical rotation of (+) and (-), is selected, and the transition metal-phosphine complex containing the selected compound as a ligand is used as a catalyst, an intended product having a desired absolute configuration in asymmetric synthesis can be obtained in a high optical purity.

Further, when rhodium is used as a transition metal by way of example, the same result as described above can be obtained even when a mixture obtained by adding an excess amount of the phosphine compound to the rhodium-phosphine complex, or mixing 2-4 equivalent weights, based on rhodium, of the phosphine compound (1) of this invention with a catalyst precursor such as commercially-available [Rh(CO)₂(acac)] is used as a catalyst.

Furthermore, if a rhodium carbonyl cluster such as Rh₆(CO)₁₆ or Rh₄(CO)₁₂ is used as a catalyst precursor, a useful catalyst is provided. Besides, it may be permissible to react Rhₙ(CO)ₙ with a halogen in advance and combine the resultant product with the phosphine compound.

The transition metal-phosphine complex is generally a mononuclear complex in which one transition metal atom is contained in the complex. However, there may be polynuclear complexes containing 2 or more transition metal atoms therein in some cases.

The present invention will hereinafter be described in detail by the following examples. However, it should be borne in mind that this invention is not limited to and by these examples only.

Incidentally, the analytical values in the examples were obtained by using the following instruments:
- Nuclear magnetic resonance spectrum (NMR):: EX-270 (manufactured by JEOL, Ltd.)

- Internal standard substance ¹H:: tetramethylsilane
- External standard substance ³¹P:: 85% phosphoric acid
- Optical purity:: GC-15A gas chromatograph (manufactured by Shimadzu Corporation)
Column: chiral capillary column, CHROMPACK β-236M
- Chemical purity:: HITACHI 263-30 gas chromatograph (manufactured by Hitachi, Ltd.)
- Optical rotation:: DIP-4 360 (manufactured by Japan Spectroscopic Co., Ltd.)

Besides, the abbreviations of the makers of reagents used in the examples have the following meanings:
- Wako:: Wako Pure Chemical Industries, Ltd.
- Tokyo:: Tokyo Kasei Kogyo Co., Ltd.
- Ald:: Aldrich Japan Ink Co., Inc.
- NACALAI:: NACALAI TESQUE INC.

### Example 1:

### Synthesis of (R)-2-diphenylphosphino-1,1'-binaphthalene-2'-yloxy((S)-1,1'-binaphthalene-2,2'-diyldioxy)phosphine:

(1) Synthesis of (R)-2,2'-bis(trifluoromethanesulfonyloxy)-1,1'-binaphthyl:
   In a 100-ml flask, 5 g (17.4 mmol) of (R)-1,1'-bi-2-naphthol (product of Mitsubishi Gas Chemical Company, Inc.), 5.58 g (52.2 mmol) of 2,6-lutidine (Tokyo) and 0.955 g (7.83 mmol) of 4-dimethylaminopyridine (Wako) were dissolved in 26 ml of methylene chloride, to which 14.7 g (52.2 mmol) of trifluoromethanesulfonic anhydride (Wako) was added at 0°C to react them for 23 hours at room temperature with stirring. After completion of the reaction, the solvent was distilled off, and the residue was then purified by column chromatography on silica gel making use of methylene chloride as a developing solvent to obtain 9.56 g (yield: 100%) of (R)-2,2'-bis(trifluoromethanesulfonyloxy)-1,1'-binaphthyl.
(2) Synthesis of (R)-2-diphenylphosphinyl-2'-trifluoromethanesulfonyloxy-1,1'-binaphthyl:
   In a 100-ml 4-necked flask, 2.74 g (4.98 mmol) of (R)-2,2'-bis(trifluoromethanesulfonyloxy)-1,1'-binaphthyl prepared in the step (1) and 1.99 g (9.82 mmol) of diphenylphosphine oxide (Tokyo) were dissolved in 20 ml of dimethyl sulfoxide in an argon stream, to which 110 mg (0.491 mmol) of palladium acetate, 203 mg (0.491 mmol) of 1,3-bis(diphenylphosphino)propane, 5.1 ml of ethyldiisopropylamine (Ald) and 33 mg (0.491 mmol) of sodium formate (NACALAI) were added to stir them for 20 minutes at room temperature. Thereafter, the resultant solution was stirred for 19 hours at 90°C and then cooled back to room temperature. The solution was added with 250 ml of ether and 150 ml of water, and the mixture was stirred to separate an organic layer and an aqueous layer. After the separation, the organic layer was washed four times with 125 ml of water, twice with 125 ml of 5% diluted hydrochloric acid, twice with 50 ml of water, once with 125 ml of a saturated aqueous solution of sodium hydrogencarbonate and lastly with 125 ml of a brine. The thus-washed organic layer was dried on anhydrous magnesium sulfate, and the solvent was distilled off. The residue was then purified by column chromatography on silica gel making use of a 3:1 (solvent ratio, the same shall apply hereinafter) mixture of toluene and acetonitrile as a developing solvent, thereby obtaining 2.51 g (yield: 83%) of the title compound.
(3) Synthesis of (R)-2-diphenylphosphino-2'-hydroxy-1,1'-binaphthyl:
   In a 50-ml 3-necked flask, 400 mg (0.664 mmol) of (R)-2-diphenylphosphinyl-2'-trifluoromethanesulfonyloxy-1,1'-binaphthyl prepared in the step (2) was dissolved in 22 ml of xylene, to which 1.21 g (12 mmol) of triethyl-amine (Wako) and 1.62 g (12 mmol) of trichlorosilane (Ald) were added. The resulting mixture was stirred for 17 hours at 120°C. After the reaction mixture was cooled back to room temperature, 4.4 ml of 35% aqueous sodium hydroxide was carefully added thereto, and the mixture was stirred further for 2 hours, followed by separation of an organic layer and an aqueous layer. After the separation, the organic layer was washed twice with 30 ml of a brine and then dried on anhydrous magnesium sulfate, and the solvent was distilled off under reduced pressure, thereby obtaining a crude product. The crude product was dissolved in 7 ml of tetrahydrofuran, to which a solution of 335 mg (7.98 mmol) of lithium hydroxide (Wako) in 2.4 ml of water was added. The resulting mixture was stirred for 15 hours at room temperature, to which 50 ml of ether and 15 ml of 5% diluted hydrochloric acid were added to separate an organic layer and an aqueous layer. After the organic layer was washed twice with water, it was dried on anhydrous magnesium sulfate, and the solvent was distilled off. The residue was then purified by column chromatography on silica gel making use of a 5:1 mixture of hexane and ethyl acetate as a developing solvent, thereby obtaining 153 mg (yield: 51%) of the title compound.
(4) Synthesis of (S)-1,1'-binaphthalene-2,2'-diyldioxychlorophosphine:
   A 50-ml flask equipped with a reflux condenser was charged with 4.94 g (17.3 mmol) of (S)-1,1'-bi-2-naphthol (product of Mitsubishi Gas Chemical Company, Inc.) and 113 g (0.83 mol) of phosphorus trichloride (Wako) in an argon stream to reflux the mixture for 4 hours. Thereafter, the reaction mixture was cooled back to room temperature, and unreacted phosphorus trichloride was distilled off under reduced pressure, thereby obtaining 5.56 g (yield: 92%) of the title compound as white crystals.
(5) Synthesis of (R)-2-diphenylphosphino-1,1'-binaphthalene-2'-yloxy((S)-1,1'-binaphthalene-2,2'-diyldioxy)phosphine:

In a 100-ml flask, 430 mg (0.946 mmol) of (R)-2-diphenylphosphino-2'-hydroxy-1,1'-binaphthyl prepared in the step (3) and 662 mg (1.89 mmol) of (S)-1,1'-binaphthalene-2,2'-diyldioxychlorophosphine prepared in the step (4) were dissolved in 30 ml of ether, to which 191 mg (1.89 mmol) of triethylamine was added at 0°C. After the mixture was stirred for 15 hours at room temperature, 20 ml of water was added to stop the reaction. After separating an organic layer and an aqueous layer, the organic layer was dried on anhydrous magnesium sulfate, and the solvent was distilled off, thereby obtaining a crude product. The crude product was purified by column chromatography on silica gel making use of a 1:1 mixture of hexane and dichloromethane as a developing solvent, thereby obtaining 712 mg (yield: 98%) of the title compound, (R)-2-diphenylphosphino-1,1'-binaphthalene-2'-yloxy-((S)-1,1'-binaphthalene-2,2'-diyldioxy)phosphine [hereinafter called "(R,S)-BINAPHOS"] as white crystals. ³¹P-NMR (CDCl₃) δ: -14.6(d,J=29.0Hz), 144.7(d,J=29.0Hz).

### Example 2:

### Synthesis of (R)-2-(diphenylphosphino)-1,1'-binaphthalene-2'-yloxy((R)-1,1'-binaphthalene-2,2'-diyldioxy)phosphine:

A 100-ml flask was charged with 30 ml of ether, to which 209 mg (0.46 mmol) of (R)-2-diphenylphosphino-2'-hydroxy-1,1'-binaphthyl prepared in the step (3) of Example 1 and 322 mg (0.92 mmol) of (R)-1,1'-binaphthalene-2,2'-diyldioxychlorophosphine were added to dissolve them in ether. To the solution, 191 mg (1.89 mmol) of triethylamine was added at 0°C. Thereafter, the same procedure as in Example 1 was followed to obtain 353 mg (yield: 96.3%) of the title compound, (R)-2-(diphenylphosphino)-1,1'-binaphthalene-2'-yloxy((R)-1,1'-binaphthalene-2,2'-diyldioxy)phosphine [hereinafter called "(R,R)-BINAPHOS"] in the form of a pale yellow solid.
³¹P-NMR (CDCl₃) δ: -18.0(d,J=9.2Hz), 140.5(d,J=9.2Hz).

### Example 3:

### Synthesis of (R)-2-(diphenylphosphino)-1,1'-binaphthalene-2'-yloxy(diphenoxy)phosphine:

The same procedure as in the step (5) of Example 1 was followed except that chlorodiphenoxyphosphine was used in place of (S)-1,1'-binaphthalene-2,2'-diyldioxychlorophosphine, thereby obtaining the title compound, (R)-2-(diphenylphosphino)-1,1'-binaphthalene-2'-yloxy(diphenoxy)phosphine [hereinafter called "(R)-Ph-BINAPHOs"] in the form of a pale yellow solid at a yield of 77%.
³¹P-NMR (CDCl₃) δ: -10.6(d,J=13.7Hz), 129.2(d,J=13.7Hz).

### Example 4:

### Synthesis of (R)-2-(di-(3,5-xylyl)phosphino)-1,1'-binaphthalene-2'-yloxy((S)-1,1'-binaphthalene-2,2'-diyldioxy)phosphine:

The same procedure as in the step (5) of Example 1 was followed except that (R)-2-(di-(3,5-xylyl)phosphino)-2'-hydroxy-1,1'-binaphthyl was used in place of (R)-2-diphenylphosphino-2'-hydroxy-1,1'-binaphthyl, thereby obtaining the title compound, (R)-2-(di-(3,5-xylyl)phosphino)-1,1'-binaphthalene-2'-yloxy((S)-1,1'-binaphthalene-2,2'-diyldioxy)phosphine [hereinafter called "(R,S)-Me-BINAPHOS"] in the form of a pale yellow solid at a yield of 98%.
³¹P-NMR (CDCl₃) δ: -12.4(d,J=32.0Hz), 145.5(d).

### Example 5:

### Synthesis of Rh((R)-Ph-BINAPHOS)(acac):

A 20-ml Schlenk's tube was charged 7 mg (0.025 mmol) of (R)-Ph-BINAPHOS prepared in Example 3 and 6.5 mg (0.025 ml) of Rh(CO)₂(acac) (acac means acetylacetonato, the same shall apply hereinafter) (product of Ald) to dissolve them in 5 ml of methylene chloride. After the reaction mixture was stirred for 5 minutes at room temperature, the solvent was distilled off under reduced pressure to obtain 22 mg (yield: 100%) of Rh((R)-Ph-BINAPHOS)(acac) as a yellow solid.
- ³¹P-NMR (CDCl₃) δ:: 51.1 (dd,Jₚ₋ₚ=82.4Hz,J_{Rh-p}=174.0Hz), 138.8(dd,J_{Rh-p}=328.1Hz).

### Example 6:

### Synthesis of Rh((R,S)-BINAPHOS)(acac):

The same procedure as in Example 5 was followed except that (R,S)-BINAPHOS prepared in Example 1 was used in place of (R)-Ph-BINAPHOS used in Example 5, thereby obtaining 63 mg (yield: 99%) of Rh((R,S)-BINAPHOS)(acac).
- ³¹P-NMR (CDCl₃) δ:: 48.3(dd,Jₚ₋ₚ=83.9Hz,J_{Rh-p}=174,0Hz), 161.8(dd,J_{Rh-p}=331.1Hz).

### Example 7:

### Synthesis of Rh((R,R)-BINAPHOS)(acac):

The same procedure as in Example 5 was followed except that (R,R)-BINAPHOS prepared in Example 2 was used in place of (R)-Ph-BINAPHOS used in Example 5, thereby obtaining 63 mg (yield: 99%) of Rh((R,R)-BINAPHOS)(acac).
- ³¹P-NMR (CDCl₃) δ:: 51.9(dd,Jₚ₋ₚ=80.8Hz,J_{Rh-p}=178.4Hz), 152.5(dd,J_{Rh-p}=335.1Hz).

### Example 8:

### Synthesis of Rh((R,S)-Me-BINAPHOS)(acac):

The same procedure as in Example 5 was followed except that (R,S)-Me-BINAPHOS prepared in Example 4 was used in place of (R)-Ph-BINAPHOS used in Example 5, thereby obtaining 37 mg (yield: 99%) of Rh((R,S)-Me-BINAPHOS)(acac).
- ³¹P-NMR (CDCl₃) δ:: 48.8(dd,Jₚ₋ₚ=82.4Hz,J_{Rh-p}=172.4Hz), 160.9(dd,J_{Rh-p}=332.6Hz).

### Referential Example 1:

### Asymmetric hydroformylation reaction of vinyl acetate:

A 50-ml autoclave was charged with 1.301 g (15.13 mmol) of vinyl acetate (Wako), 9.8 mg (0.0378 mmol) of Rh(CO)₂(acac) as a catalyst precursor, 58 mg (0.0755 mmol) of (R,S)-BINAPHOS as an optically active phosphine and 14 ml of benzene. The contents were stirred at 60°C for 112 hours under a hydrogen pressure of 50 atm and a carbon monoxide pressure of 50 atm. The determination of conversion of vinyl acetate and the analysis of the reaction products were conducted by gas chromatography (10% SE-30 on Chromosorb W 0.18-0.149 mm (80-100 mesh) packed in a 5 mm x 2 m glass column), and the optical purities of the products were determined by optically active gas chromatography (Astec Chiraldex B-PH). As a result, it was found that compounds produced by this reaction were a mixture of 90% of 2-acetoxypropanal and 10% of 3-acetoxypropanal, and the conversion was 98%. 2-Acetoxypropanal was then distilled to determine its optical purity. As a result, it was found to be 90% ee. This compound was then caused to undergo Jones oxidation to derive 2-acetoxypropionic acid. Its optical rotation was measured. As a result, it was confirmed that the compound is (S)-(-)-2-acetoxypropionic acid.

### Referential Example 2:

### Asymmetric hydroformylation reaction of vinyl acetate:

The same procedure as in Referential Example 1 was followed except that Rh((R,R)-BINAPHOS)(acac) was used as a catalyst, and the reaction was conducted for 37 hours at 50°C, thereby obtaining a mixture of 92% of 2-acetoxypropanal and 8% of 3-acetoxypropanal. The conversion was 46%. The optical purity of 2-acetoxypropanal after distilled was determined in the same manner as in Referential Example 1, and was found to be 73% ee. This compound was then caused to undergo Jones oxidation to derive 2-acetoxypropionic acid. Its optical rotation was measured. As a result, it was confirmed that the compound is (S)-(-)-2-acetoxypropionic acid.

### Referential Example 3:

### Asymmetric hydroformylation reaction of styrene:

A 50-ml autoclave was charged with 3.06 g (29.4 mmol) of styrene (NACALAI), 3.7 mg (0.0143 mmol) of Rh(CO)₂(acac), 44 mg (0.0573 mmol) of (S,R)-BINAPHOS and 1 ml of benzene-d₆. The contents were stirred at 60°C for 43 hours under a hydrogen pressure of 50 atm and a carbon monoxide pressure of 50 atm. After completion of the reaction, the reaction mixture was cooled back to room temperature, and the gases under pressure were removed. Thereafter, the reaction mixture was directly analyzed by ¹H-NMR. As a result, it was found that the conversion of styrene was at least 99%, and a mixture of 88% of 2-phenylpropanal and 12 % of 3-phenylpropanal as reaction products was obtained in an amount of 1.273 g. No other products were observed. 2-Phenylpropionic acid was derived in the same manner as in Referential Example 1 to determine its optical purity. As a result, it was confirmed that the compound is (S)-(+)-2-phenylpropionic acid having an optical purity of 94% ee.

## Claims

1. A phosphine compound represented by the following formula (1): 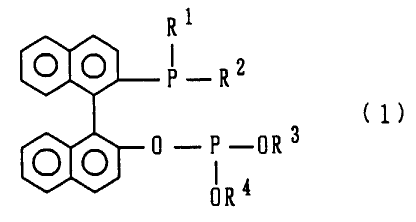 wherein R¹ and R² are identical with or different from each other and mean individually a phenyl group which may be substituted by a halogen atom or lower alkyl group, or denote together a divalent hydrocarbon group, R³ and R⁴ are identical with or different from each other and mean individually a lower alkyl group or a phenyl group which may be substituted by a halogen atom, lower alkyl group or lower alkoxy group, or denote together a divalent hydrocarbon group.

2. A transition metal-phosphine complex containing, as a ligand, the phosphine compound according to Claim 1.

3. The transition metal-phosphine complex according to Claim 2, wherein the transition metal is selected from rhodium, ruthenium, palladium, iridium, platinum, cobalt, nickel and titanium.

4. A rhodium-phosphine complex represented by the following formula (2-a) or (2-b):
[Rh(L)(Y)(X)] (2-a)
[Rh(L)(Y)](X) (2-b)
wherein L means the phosphine compound according to Claim 1, Y denotes carbon monoxide, a monodentate or bidentate neutral ligand having lower coordinating strength than that of carbon monoxide, olefin or diene, and X stands for a hydrogen atom, monovalent anion or triphenylphosphine, or X and Y mean together a β-diketonate.

5. The rhodium-phosphine complex according to Claim 4, wherein in the formula (2-a) or (2-b), the combination of X and Y is selected from the group consisting of the following combinations:
(a) Y = CO and X = halogen atom or cyano group;
(b) Y = cycloocta-1,5-diene and X = halogen atom, ClO₄, BF₄, PF₆, CH₃COO or cyano group;
(c) Y = norbornadiene and X = halogen atom, ClO₄, BF₄, PF₆, CH₃COO or cyano group;
(d) Y = (CO)₂ and X = hydrogen atom or (triphenylphosphine)₂;
(e) Y = (CO)₃ and X = triphenylphosphine;
(f) Y = ethylene and X = halogen atom or cyano group;
(g) Y = pyridine and X = chlorine or bromine atom; and
(h) X and Y = together β-diketonate.

## Patentansprüche

1. Phosphinverbindung der folgenden Formel (1): 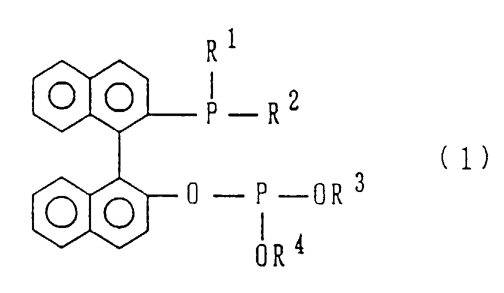 worin R¹ und R² gleich oder verschieden voneinander sein können und jeweils für eine Phenylgruppe stehen, die durch ein Halogenatom oder eine Niedrig-Alkylgruppe substituiert sein kann, oder zusammen eine zweiwertige Kohlenwasserstoffgruppe bezeichnen, R³ und R⁴ gleich oder verschieden voneinander sind und jeweils für eine Niedrig-Alkylgruppe oder eine Phenylgruppe, die durch ein Halogenatom, eine Niedrig-Alkylgruppe oder Niedrig-Alkoxygruppe substituiert sein kann, stehen oder zusammen eine zweiwertige Kohlenwasserstoffgruppe bezeichen.

2. Übergangsmetall-Phosphin-Komplex enthaltend als Ligand die Phosphinverbindung nach Anspruch 1.

3. Übergangsmetall-Phosphin-Komplex nach Anspruch 2, **dadurch gekennzeichnet**, daß das Übergangsmetall aus Rhodium, Ruthenium, Palladium, Iridium, Platin, Kobalt, Nickel und Titan ausgewählt ist.

4. Rhodium-Phosphin-Komplex der folgenen Formel (2-a) oder (2-b) :
[Rh(L) (Y) (X)] (2-a)
[Rh(L) (Y)] (X) (2-b)
worin L die Phosphinverbindung nach Anspruch 1 bezeichnet, Y für Kohlenmonoxid, einen einzähnigen oder zweizähnigen neutralen Liganden mit niedrigerer Koordinationsstärke als die von Kohlenmonoxid, ein Olefin oder Dien steht und X für ein Wasserstoffatom, ein einwertiges Anion oder Triphenylphosphin steht oder X und Y zusammen ein β-Diketonat bezeichnen.

5. Rhodium-Phosphin-Komplex nach Anspruch 4, **dadurch gekennzeichnet**, daß in den Formeln (2-a) oder (2-b) die Kombination von X und Y aus der Gruppe bestehend aus den folgenden Kombinationen ausgewählt ist:
(a) Y = CO und X = ein Halogenatom oder eine Cyano-Gruppe;
(b) Y = Cycloocta-1,5-dien und X = ein Halogenatom, ClO₄, BF₄, PF₆, CH₃COO oder eine Cyano-Gruppe;
(c) Y = Norbornadien und X = ein Halogenatom, ClO₄, BF₄, PF₆, CH₃COO oder eine Cyano-Gruppe;
(d) Y = (CO)₂ und X = ein Wasserstoffatom oder (Triphenylphosphin)₂;
(e) Y = (CO)₃ und X = Triphenylphosphin;
(f) Y = Ethylen und X = ein Halogenatom oder eine Cyano-Gruppe;
(g) Y = Pyridin und X = ein Chlor- oder Bromatom; und
(h) X und Y bezeichnen zusammen ein β-Diketonat.

## Revendications

1. Composé de phosphine représenté par la formule suivante (1): 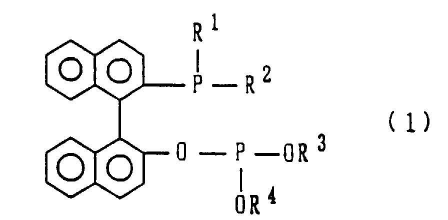 dans laquelle R¹ et R² sont identiques ou différents l'un de l'autre et représentent individuellement un groupe phényle qui peut être substitué par un atome d'halogène ou un groupe alkyle inférieur ou représentent ensemble un groupe hydrocarboné divalent, R³ et R⁴ sont identiques ou différents l'un de l'autre et représentent individuellement un groupe alkyle inférieur ou un groupe phényle qui peut être substitué par un atome d'halogène un groupe alkyle inférieur ou un groupe alcoxy inférieur ou représentent ensemble un groupe hydrocarboné divalent.

2. Complexe métal de transition-phosphine contenant en tant que ligand, le composé de phosphine selon la revendication 1.

3. Complexe métal de transition-phosphine selon la revendication 2, dans lequel le métal de transition est choisi parmi le rhodium, le ruthénium, le palladium, l'iridium, le platine, le cobalt, le nickel et le titane.

4. Complexe rhodium-phosphine représenté par les formules suivantes (2-a) ou (2-b) :
[Rh(L)(Y)(X)] (2-a)
[Rh(L)(Y)](X) (2-b)
dans lesquelles L représente le composé de phosphine selon la revendication 1, Y représente le monoxyde de carbone, un ligand monodentate ou bidentate neutre ayant une force de coordination plus faible que celle du monoxyde de carbone, une oléfine ou un diène et X représente un atome d'hydrogène, un anion monovalent ou la triphénylphosphine et X et Y représentent ensemble un β-dicétonate

5. Complexe rhodium-phosphine selon la revendication 4, dans lequel dans la formule (2-a) ou (2-b), la combinaison de X et de Y est choisie au sein du groupe comprenant les combinaisons suivantes :
(a) Y = CO et X = atome d'halogène ou groupe cyano ;
(b) Y = cycloocta-1,5-diène et X = atome d'halogène, ClO₄, BF₄, PF₆, CH₃COO ou groupe cyano ;
(c) Y = norbornadiène et X = atome d'halogène, ClO₄, BF_{4,} PF₆, CH₃COO ou groupe cyano ;
(d) Y = (CO)₂ et X = atome d'hydrogène ou (triphénylphosphine)₂;
(e) Y = (CO)₃ et X = triphénylphosphine ;
(f) Y = éthylène et X = atome d'halogène ou groupe cyano ;
(g) Y = pyridine et X = atome de chlore ou de brome ; et
(h) X et Y ensemble = β-dicétonate.
